# EUROPEAN PATENT SPECIFICATION

(11) **EP 2 904 113 B1**
(45) Date of publication and mention of the grant of the patent: **26.02.2020**
(21) Application number: 13780321.9
(22) Date of filing: 07.10.2013
(51) Int. Cl.: C12Q 1/6855, C12Q 1/6806

(54) **HIGH-THROUGHPUT GENOTYPING BY SEQUENCING LOW AMOUNTS OF GENETIC MATERIAL**
GENOTYPISIERUNG MIT HOHEM DURCHSATZ DURCH SEQUENZIERUNG GERINGER MENGEN EINES GENETISCHEN MATERIALS
GÉNOTYPAGE À HAUT RENDEMENT PAR SÉQUENÇAGE DE FAIBLES QUANTITÉS DE MATÉRIEL GÉNÉTIQUE

(30) Priority: 05.10.2012 GB 201217888
(43) Date of publication of application: 12.08.2015
(60) Divisional application: 19219353.0
(73) Proprietor: Katholieke Universiteit Leuven K.U. Leuven R&D, 3000 Leuven (BE)
(72) Inventor: VERMEESCH, Joris, B-3020 Veltem-Beisem (BE); VOET, Thierry, B-3001 Heverlee (BE); HANNES, Femke, B-3080 Tervuren (BE); VAN HOUDT, Jeroen, B-3190 Boortmeerbeek (BE); MAES, Gregory, B-3010 Kessel-Lo (BE)
(74) Representative: Gevers Patents
(86) International application number: PCT/EP2013/070858
(87) International publication number: WO 2014/053664

(56) References cited:
- WO-A1-2013/078019
- WO-A1-2013/106737
- WO-A2-2004/081183
- US-B1- 6 395 887
- DENIS D ET AL: "Whole Genome Amplification (WGA) and Short Tandem Repeat Analysis (STR) of Single Blastomeres for Embryo Fingerprinting and Embryo Genomics", FERTILITY AND STERILITY, ELSEVIER SCIENCE INC, NEW YORK, NY, USA, vol. 84, 1 September 2005 (2005-09-01), page S338, XP027698829, ISSN: 0015-0282 [retrieved on 2005-09-01]
- KLEIN C A ET AL: "Comparative genomic hybridization loss of heterozygosity, and DNA sequence analysis of single cells", PROCEEDINGS OF THE NATIONAL ACADEMY OF SCIENCES, NATIONAL ACADEMY OF SCIENCES, US, vol. 96, no. 8, 13 April 1999 (1999-04-13) , pages 4494-4499, XP002130910, ISSN: 0027-8424, DOI: 10.1073/PNAS.96.8.4494
- XUN XU ET AL: "Single-Cell Exome Sequencing Reveals Single-Nucleotide Mutation Characteristics of a Kidney Tumor", CELL, CELL PRESS, US, vol. 148, no. 5, 15 February 2012 (2012-02-15), pages 886-895, XP028402803, ISSN: 0092-8674, DOI: 10.1016/J.CELL.2012.02.025 [retrieved on 2012-02-17]
- ROBERT J. ELSHIRE ET AL: "A Robust, Simple Genotyping-by-Sequencing (GBS) Approach for High Diversity Species", PLOS ONE, vol. 6, no. 5, 1 January 2011 (2011-01-01) , page e19379, XP55033060, ISSN: 1932-6203, DOI: 10.1371/journal.pone.0019379
- R. W. K. Chiu ET AL: "Noninvasive prenatal diagnosis of fetal chromosomal aneuploidy by massively parallel genomic sequencing of DNA in maternal plasma", Proceedings of the National Academy of Sciences, vol. 105, no. 51, 23 December 2008 (2008-12-23), pages 20458-20463, XP55284693, US ISSN: 0027-8424, DOI: 10.1073/pnas.0810641105

## Description

### Technical field

The present disclosure relates to a method and system providing a rapid discovery, validation and assessment of genetic variations or chromosomal disorders throughout the whole genome including both sex chromosomes and/or the mitochondrial genomes in samples containing low amounts of target nucleic acids, such as relatively small analytes, such as few or single cells or free-flowing tumor or fetal nucleic acids.

### Technical background

The most common form of genetic variation in the human genome is a class of genetic variation known as a single nucleotide polymorphism (SNP). SNPs are important markers in many studies that link sequence variations to phenotypic changes. Hence, the identification of SNPs also known as SNP-typing is an important tool in molecular diagnostics and aims to determine on which positions at least one of the bases differs from the reference sequence. Genotyping is the process of allele discrimination for an individual. Genotypes are typically identified using DNA extracted from thousands of cells.

In contrast to using DNA extracted from a large number of cells, more recently, technology has been developed which allows, high capacity, low cost genome-wide genotyping of small analytes such as single cells or a limited number of cells. SNP-and Geno-typing single cells or a limited number of cells are daunting tasks due to the small amount of DNA available (-7pg for a normal diploid human cell or -3.3 pg for a haploid cell). To overcome this small amount of input material, an extensive whole genome amplification (WGA) is usually performed prior to further downstream analysis. Different WGA methods have been described, and are based on either a Multiple Displacement amplification (MDA) (e.g. Genomiphi and Repli-G kit) or a PCR-based genome wide amplification method (e.g. GenomePlex). Subsequent to this amplification, successful genotyping has been achieved via "SNP chip" microarray-based platforms as known in the art. Those platforms require a substantial prior knowledge of both genome sequence and variability, and once designed are suitable only for those targeted variable nucleotide sites. This method introduces substantial ascertainment bias and inherently precludes detection of rare or population-specific variants or its use in highly diverse species.

Novel sequencing technologies enabled to assess the variation of several 10,000's of targets at a genome-wide level through high-throughput massively parallel sequencing (i.e. next-generation sequencing or NGS) that enabled fast genome-wide sequencing. NGS typically yields several orders of magnitude more data than traditional Sanger Sequencing. In order to retrieve SNP- and/or genotype data from NGS studies, extensive bioinformatic/statistical interpretation of the data is needed including algorithms for base calling and genome alignment followed by tools for SNP identification and/or genotype determination. Besides whole genome amplification, partial genome amplification (PGA) is sometimes preferred to promote enrichment of certain DNA fragments of interest (e.g. a collection of genes or exons, the mitochondrial genome, etc.). Both, whole genome and targeted amplification strategies have been reported in relation with high-throughput massive parallel sequencing efforts.

Recently, single cell sequencing was achieved from both complete genomes and capture exome libraries and, as a result, deeper insights were gained in different fields such as tumor biology and gametogenesis. Navin and colleagues developed a FACS-based method to isolate individual nuclei from different sections of a breast cancer sample and performed whole genome amplification followed by massive parallel sequencing. The WGA-products were sequenced at low coverage (∼ 0,2X), sufficient to calculate copy number variations. However, their approach disadvantageously did not allow detecting somatic base mutations in single cells. Xu et al. (Cell 2012 148(5):886-95) and Hou et al. (Cell 2012 148(5):873-85) used mouth pipetting to isolate individual cells from a solid and hematopoietic tumor. Following amplification, exome capture was performed previous to high-throughput single cell sequencing which enabled both groups to analyze the genetic landscape of somatic base mutations in complex tumors. Sequencing depths between 30X and 40X could be obtained, but the majority of single-cell exomes were sequenced to a minimum depth of 5X. In order to assess true somatic mutations within the coding regions, the putative variation was filtered according to multiple criteria including the presence of the mutation in at least 3 to 5 different single cell samples. In contrast, Wang and colleagues used a revolutionary microfluidic system to separate individual sperm cells and performed sample processing in parallel that includes whole-genome amplification to improve amplification performance. Following WGA, high-throughput whole genome sequencing analysis was performed to determine homologous recombination and gene conversion events as well as de novo mutation rates of base substitutions and chromosome aneuploidies. Only 30 to 50% of the genome was represented due to an amplification bias at a sequence coverage of 6 to 8 times. In addition, Wang et al sequenced MDAed single sperm cells at lower genome coverage in a multiplex reaction to perform aneuploidy detection. WO2012108920 provides methods for non-invasive prenatal ploidy calling. DNA from single cells or fetal DNA from plasma samples obtained from pregnant women are amplified with Specific Target Amplification (STA) using hundreds to thousands of primer pairs in a semi-nested multiplex PCR. Amplicons are sequenced to determine the ploidy state of three chromosomes. WO2007073165 A1 describes a method for preparing nucleic acid libraries for high throughput sequencing. Chiu R.W.K. et al., PNAS, vol. 105, no. 51, pp. 20458-20463, 2008) describes method for preparing a nucleic acid library from low amounts of fetal nucleic acids. Overall, § complete genome analysis of the read count information enabled the genome wide detection of large-scale copy number aberrations in the genome and multiplexing of single exome sequencing enabled to detect individual mutations. However thus far, no accurate SNP-calling has been achieved from high-throughput massive sequencing data from a single cell.

Besides the lack of a method that can achieve high-throughput massive sequencing from small analytes of samples containing a limited amount of DNA, prior art methods also carry several drawbacks. For example, prior art methods require the development and design of SNP arrays or multiplex primer sets. In each instance, these methods require a detailed knowledge of the genome, a lot of time and computing efforts and several trial-and-error runs and optimizations in order to apply the method to a new genome. Furthermore, users need to obtain expensive arrays and primers/probes and the methods take a long time to perform, often necessitating multiple days from sample to result. In addition, prior art methods do not allow a high-throughput analysis of several samples at once, as arrays do not allow for large amounts of samples to be detected at the same time and multiplex PCR analysis, such as described in WO2012108920, does not allow for increasing the numbers of assays that can be run simultaneously. In contrast, the present invention provides a straightforward method for sequencing samples containing a low amount of target DNA, which is easily transferable for application to other genomes (e.g. unsequenced or partially sequenced genomes), allows for a high-throughput analysis and the sequencing of multiple samples at once, is low in operator time and cost and doesn't necessitate expensive consumables (such as arrays or thousand of specific primer sets). Generating a reduced representation library according to the methods of the invention can be performed in about 3-6h, while next generation sequencing allows the sequencing to be performed in about 2-4h (e.g. using the ion torrent platform). Thus, results can be obtained in about 5-10h, which is much faster than prior art methods which often require multiple days. Especially in pre-implantation diagnostics, such a time reduction is a crucial advantage.

Considering the relative high cost and complexity to sequence and assemble a complete genome, several strategies have been developed that enable the rapid and cost-efficient genome wide discovery and genotyping of genetic variants (SNPs, INDELs, CNVs) from only partially sequenced genomes. Up to now, several new methods have been developed to reduce the sequencing effort and to restrict screening to a few thousand single nucleotide polymorphisms (SNPs) at a highly reduced cost compared to whole genome sequencing or biased SNP-chip analyses. These methods have been aiming at constructing reduced representation libraries (or RRLs) to reduce the complexity of the genome before sequencing, by (1) enrichment for subsets of the genome either by capturing/targeting known fragments or (2) by the removal of highly repetitive, large complex fragments by restriction enzyme digestion. Examples of the latter method include complexity reduction of polymorphic sequences (CRoPS), multiplexed shotgun genotyping, restriction-site-associated DNA sequencing (RAD-seq) and Genotyping-by-Sequencing or GBS. All methods are based on a straightforward and flexible restriction enzyme digestion and adaptor ligation, followed by deep sequencing, especially of use for those species without the reference genome.

The Genotyping-by-sequencing (GBS) approach is straightforward, quick, highly specific and reproducible, and allows to access genomic regions that are inaccessible to sequence capture approaches. In species lacking a complete genome sequence, GBS allows a reference map to be constructed during the process of sample genotyping, while genome-enabled species can greatly benefit from the additional sequence information to improve the discovery of novel polymorphisms outside exons. GBS is particularly useful, as it enables us to reduce the genomic regions queried to a scalable number of loci, typically from a few thousands to 100,000 depending on the applications envisaged.

The RAD-tag sequencing is e.g. also disclosed in EP 1885882 and CROPS technology is described in van Orsouw et al. (Plos One 2(11): e1172. doi:10.1371/journal.pone.0001172).

### Summary of the invention

A need still exists for an improved system and method for genotyping by sequencing of small analytes such as for instance a single cell, a limited number of cells or a sample containing genetic material of interest that is only available in limited amounts.

It is a general object of the present invention to provide an alternative system and method for genetic testing by sequencing of small analytes, such as single cells, dual cells, few cells or samples containing a limited amount of genetic material of interest.

It is an object of the present invention to provide an alternative system and method for genotyping and/or genetic testing by sequencing of a single cell.

It is another object of the present invention to provide an alternative system and method for genotyping and/or genetic testing by sequencing of a few cells. As further detailed hereinafter, a few cells corresponds to a sample containing up to 30 target cells, in particular one or two target cells. Alternatively, the number of cells may be based on the amount of genetic material of interest present in the sample and within the context of the present invention corresponds with a sample wherein genetic material of interest is present in an amount of 100 pg or less.

It is yet another object of the present invention to provide an alternative system and method for genotyping and/or genetic testing by sequencing of samples comprising low amounts of target nucleic acids, also referred to as the genetic material of interest.

This object is met by the method and means according to the independent claims of the present invention. The dependent claims relate to preferred embodiments.

In one aspect, the present invention provides methods for small analyte genetic testing, the method comprising following steps:
i. isolating at least one small analyte,
ii.massively parallel (genome-wide) genetic polymorphism typing by sequencing a reduced representation library of the genetic material of interest present within said small analyte,
iii. apply for variant discovery, genotyping and/or haplotyping .

The methods of the invention are particularly advantageous in procedures requiring accuracy and efficiency and outcome delivery within small time frames, such as for instance in preimplantation genetic diagnosis. Preferably the small analyte is physical matter such as genetic material or cells containing genetic material. More preferably, the analyte is an analyte used in preimplantation genetic diagnosis or screening. The analyte may be a single cell, a dual-cell, a few cells or simply low amounts of nucleic acids. Because the amount of genetic DNA obtainable from a few or single cells is limited, In some embodiments, the step of obtaining genetic material from the analyte may require amplification before sequencing.

Accordingly, the disclosure also provides methods for small analyte genetic testing, the method comprising following steps:
i. isolating at least one small analyte,
ii. amplifying DNA fragments of the genetic material present within said small analyte to form an amplification product,
iv. massively parallel (genome-wide) genetic polymorphism typing by sequencing a reduced representation library of said amplification product,
v. apply for variant discovery, genotyping and/or haplotyping

In case the analyte is a cell (single or more), the methods of the invention comprise the additional step of lysing the isolated cell for the release of nucleic acid (e.g. DNA or RNA).

Accordingly, the disclosure also provides methods for small analyte genetic testing, the method comprising following steps:
i. isolating and lysing at least one small analyte,
ii. amplifying DNA fragments of said genetic material to form an amplification product,
iv.massively parallel (genome-wide) genetic polymorphism typing by sequencing a reduced representation library of said amplification product,
v. apply for variant discovery, genotyping and/or haplotyping

Instead of a cell, the analyte may simply be small amounts of genetic material, such as for instance fetal DNA in maternal liquid (e.g. blood).

Thus, in a related aspect the disclosure provides methods for genotyping and/or haplotyping small amounts of genetic material, the method comprising following steps:
i. providing small amounts of genetic material,
ii. amplifying DNA fragments of the genetic material
iii. massively parallel (genome-wide) genetic polymorphism typing by sequencing a reduced representation library of said amplification product,
iv. apply for variant discovery, genotyping and/or haplotyping.

In particular embodiments, the disclosure provides methods for single cell genotyping and/or haplotyping, the method comprising following steps:
i. isolating and lysing the single cell,
ii. amplifying DNA fragments of the single cell,
iii. massively parallel (genome-wide) genetic polymorphism typing (genotyping) by deep sequencing a reduced representation library of said amplification product,
iv. a pipeline for variant discovery, genotyping and/or haplotyping.

In another particular embodiment the disclosure provides methods for dual cell genotyping and/or haplotyping, the method comprising following steps:
i. isolating and lysing two cells,
ii. for each cell, amplifying the single cell DNA fragments,
iii. for each cell massively parallel (genome-wide) genetic polymorphism typing (genotyping) by deep sequencing a reduced representation library of said single cell amplification product,
iv. generating a virtual genotype consisting of genetic polymorphism calls concordant between the two separately genotypes single cells,
v. reconstructing the haplotype of said virtual genotype) or a selection of said virtual genotype,
iv. a pipeline for variant discovery, genotyping and/or haplotyping.

In an alternative embodiment, the disclosure provides methods for genotyping and/or haplotyping at least one cell, the method comprising following steps:
i. isolating and lysing the at least one cell,
ii. amplifying DNA fragments of the least one cell,
iii. massively parallel (genome-wide) genetic polymorphism typing (genotyping) by deep sequencing a reduced representation library of said amplification product,
iv. a pipeline for variant discovery, genotyping and/or haplotyping.

In yet another particular embodiment, the present invention provides methods for analysis of target nucleic acids, the method comprising the following steps:
i. providing a sample wherein target nucleic acids are present in a low amount,
ii. generating a reduced representation library of said target nucleic acids,
iii. massively parallel sequencing said reduced representation library, and
iv. identifying variants in said target nucleic acids by analyzing results obtained by said sequencing.

In a particular embodiment, generating a reduced representation library further comprises whole genome amplification. Therefore, in a particular embodiment, the present invention provides a method for analysis of target nucleic acids, the method comprising the following steps:
i. providing a sample wherein target nucleic acids are present in a low amount,
ii. optionally amplifying said target nucleic acids,
iii. generating a reduced representation library of said target nucleic acids,
iv. massively parallel sequencing said reduced representation library, and
v. identifying variants in said target nucleic acids by analyzing results obtained by said sequencing.

In a preferred embodiment, the methods of the present invention are applicable on a genome-wide scale. Therefore, in a particular embodiment, the present invention provides a method for genome-wide analysis of target nucleic acids, the method comprising the following steps:
i. providing a sample wherein target nucleic acids are present in a low amount,
ii. optionally amplifying said target nucleic acids,
iii. generating a genome-wide reduced representation library of said target nucleic acids,
iv. massively parallel sequencing said reduced representation library, and
v. genome-wide identifying variants in said target nucleic acids by analyzing results obtained by said sequencing.

In a particular embodiment, said target nucleic acids are amplified prior to the generation of a reduced representation library. In another particular embodiment, the generation of a reduced representation library comprises amplifying a subset of said target nucleic acids.

In preferred embodiments amplifying is performed on the whole genome. Whole Genome Amplification (WGA) amplifies single nucleotide polymorphisms (SNPs), mutations and copy number variations across the entire genome for analysis. Several techniques of WGA have been described including ligation-mediated PCR (LM-PCR), degenerate oligonucleotide primer PCR (DOP-PCR), and multiple displacement amplification (MDA). In a particular embodiment, the methods of the invention comprise whole genome amplification (WGA) or target nucleic acids.

In other preferred embodiments of the disclosure amplifying may be performed using whole-genome multiple displacement amplification or any whole-genome amplification method.

In preferred embodiments of the invention the method further may comprise constructing a reduced representation library of the amplification product for massively parallel sequencing and subsequent apply for variant discovery, genotyping and/or haplotyping using bioinformatics and statistical means.

In a particular embodiment, the reduced representation library is produced by a method comprising fragmenting said target nucleic acids, ligating adaptors to said fragments and selecting a subset of said adaptor-ligated fragments. In a further particular embodiment, fragmenting said target nucleic acids comprises digesting said target nucleic acids with one or more restriction enzymes. In another further embodiment, fragmenting said target nucleic acids comprises physical shearing, for example using ultrasound. One more different adaptors may be used for ligation to said fragments. In a particular embodiment, said adaptor-ligated fragments are further amplified using primers that anneal to said adaptors. In another particular embodiment, selecting a subset of adaptor-ligated fragments is based on the size of said fragments. In a further particular embodiment, selecting a subset of adaptor-ligated fragments comprises size-selection by PCR-amplification. In another embodiment, size-selection is performed during isolation of the reduced representation library, e.g. using PCR purification methods.

Therefore, in a preferred embodiment, the present invention provides a method for analysis of target nucleic acids, the method comprising the following steps:
i. providing a sample wherein target nucleic acids are present in a low amount,
ii. generating a reduced representation library of said target nucleic acids by a method comprising
   - fragmenting said target nucleic acids;
   - ligating adaptors to said fragments; and
   - selecting a subset of said adaptor-ligated fragments,
iii. massively parallel sequencing said reduced representation library, and
iv. identifying variants in said target nucleic acids by analyzing results obtained by said sequencing.

In a particular embodiment, the methods of the present invention further comprise constructing a genotype and/or haplotype based on identified variants in said target nucleic acids. In another particular embodiment, the methods of the invention further comprise identifying a genetic aberration in said sample based on identified variants in said target nucleic acids.

In another particular embodiment, selecting a subset of adaptor-ligated fragments comprises an amplification reaction using a selective primer. In particular, said selective primer contains from 1 to 5 selective nucleotides at its 3' end. Amplification using the selective primer only amplifies a subset of said adaptor-ligated fragments, namely those to which the selective primer hybridizes with sufficient stringency to allow its elongation. In another particular embodiment, said selective primer contains from 1 to 3, more in particular 2 selective nucleotides at the 3' end. In another particular embodiment, said selective primer contains an adaptor region and a selective region. Said adaptor region hybridizes to the adaptor in single-stranded adaptor-ligated fragments, while said selective region consists of selective nucleotides. Said selective nucleotides hybridizing with nucleotides present in the fragment between the adaptors. In a particular embodiment, said selective primer comprises from 5' to 3' an adaptor region, an optional linker region and a selective region, wherein said adaptor region and selective region are as described above. Said linker region comprising from 1 to 50, in particular 1-25, more in particular 1-10 nucleotides.

Preferably the reduced representation library of the genetic material amplification product or the at least one cell's amplification product is produced by restriction digestion using at least one or a combination of restriction enzymes and subsequent adaptor ligation and size-selection by PCR-amplification, or any sequence library reduction method known in the art. The generation of a reduced representation library using fragmentation or restriction digestion is especially preferred, as it is a straightforward method that does not require the design and use of specific primers and/or probes. The reduced representation method can be applied easily to different genomes, even when having limited information about these genomes, without the need for complex (primer/probe/array) design considerations and reducing bias inherent in prior art methods.

In another particular embodiment of the disclosure the sequence library reduction method may further comprise exon capture. Preferably the exon capture can be performed using any of exome sequencing methods know in the art or any targeted exome capture methods in the art. The latter can be an efficient strategy to selectively sequence the coding regions of the genome as a cheaper but still effective alternative to whole genome sequencing. Exons are short, functionally important sequences of DNA which represent the regions in genes that are translated into protein and the untranslated region (UTR) flanking them. UTRs are usually not included in exome studies. In the human genome there are about 180,000 exons: these constitute about 1% of the human genome, which translates to about 30 megabases (Mb) in length. It is estimated that the protein coding regions of the human genome constitute about 85% of the disease-causing mutations. In a preferred embodiment, the methods of the invention do not comprise exon capture. In another particular embodiment, the methods of the disclosure do not comprise bisulfite conversion.

It has been found that the generation of a reduced representation library in combination with sequencing allows for larger sequencing depths, while maintaining genome-wide information. The amount of library reduction can be chosen by the skilled person dependent on the number of variants one wants to identify, the sequencing depth one wants to obtain for these variants, the available sequencing infrastructure and the sequencing costs. For example, very large reductions can be obtained by using stringent fragment selection. Such a strongly reduced representation library can be sequenced at high depths with minimal efforts. Nonetheless, they provide a genome-wide picture of variants, which can be used for e.g. ploidy calling or haplotype determination. In instances where genome-wide information should be available at a higher resolution, the skilled person can apply a less stringent reduction of the sequencing library. In a particular embodiment, the library reduction reduces the complexity at least 5 times. In another embodiment at least 10 times, in particular at least 50 times, more in particular at least 100 times. In yet another particular embodiment, the complexity is reduced at least 200 times, in particular at least 500 times, more in particular at least 1000 times. For example, a complexity reduction of 100 times means that the reduced representation library provides fragments covering about 1% of the genome, thereby strongly reducing sequencing efforts and allowing for larger sequencing depths of the remaining fragments. Nonetheless, as these fragments are scattered throughout the genome, the methods of the present invention provide genome-wide variant information.

In other preferred embodiments the method further may comprise the step of deep sequencing of the reduced representation library. The latter advantageously assures that each variant position is sampled with high redundancy. The robust approach to sequencing the reduced representation library advantageously has the potential to be clinically relevant in genetic diagnosis due to current understanding of functional consequences in sequence variation. The goal of this approach is to identify the functional variation that is responsible for both mendelian and common diseases, e.g. such as Miller syndrome and Alzheimer's disease, without the high costs associated with whole-genome sequencing while maintaining high coverage in sequence depth.

In other preferred embodiments the pipeline for variant calling or application for variant discovery, genotyping and/or haplotyping may be based on the detection of variant allele frequencies, in the sequence reads, that are discriminated from sequencing and/or amplification inconsistencies using a pipeline of sequence alignment, bioinformatics and statistics.

In preferred embodiments the variant allele frequencies may be rare variant allele frequencies.

Preferably using a pipeline of sequence alignment is performed using a reference genome. In a particular embodiment, the methods of the present invention further comprise comparing identified variants to a reference sequence, in particular a reference genome.

In other preferred embodiments the method may further comprise the step of inferring genotype calls from detected variant allele frequencies.

In preferred embodiments the method further may comprise haplotype assessment and/or prediction of the at least one cell's genotype, preferably of a single or dual cell's genotype.

Preferably the amplifying amplifies only part of the genome.

In other preferred embodiments the partial genome amplifying (PGA) is performed using multiple displacement amplification or any DNA-amplification method. Preferably any of PicoPlex, GenomePlex, SurePlex and/or AmpliOne. Alternatives which can be used may include any DOP-PCR, PEP-PCR, ligation- mediated PCR, and/or alu-PCR whole genome amplification methods known in the art.

In other preferred embodiments the method may further comprise the construction of a library of the PGA-product for massively parallel sequencing and subsequent genotyping and/or haplotyping using bioinformatics and statistical means. Preferably said library is a reduced representation library.

Preferably the reduced representation library of the small analyte's PGA-product is produced by restriction digestion using one or a combination of restriction enzymes and subsequent adaptor ligation and size-selection by PCR-amplification, or any sequence library production method with or without further representation reduction method.

In other preferred embodiments the method further may comprise the step of deep sequencing of the reduced representation library to assure that each variant position is sampled with high redundancy.

In preferred embodiments of the invention the pipeline for variant calling is based on the detection of variant allele frequencies in the sequence reads that can be discriminated from sequencing and/or amplification artifacts using a pipeline of sequence alignment, bioinformatics and statistics.

Preferably the variant allele frequencies are rare variant allele frequencies.

In preferred embodiments of the disclosure using a pipeline of sequence alignment is performed using a reference genome.

In other preferred embodiments of the invention the method further may comprise the step of inferring genotype calls from detected variant allele frequencies.

In preferred embodiments of the invention the method further may comprise haplotype assessment or prediction of the at least one cell's, preferably a single cell's, genotype.

In other preferred embodiments of the disclosure amplifying may involve immediate reduced representation sequence library production from the DNA present in the at least one cell's, preferably a single cell's, lysate. Consequently, in particular embodiments herein provided, the small analyte is either a single cell or the DNA present within said single cell or a lysate thereof.

In preferred embodiments of the invention following lysis, the at least one, preferably a single, cell's DNA is preferably immediately digested by one or a combination of restriction enzymes and subsequent adaptor ligation and size-selection by PCR-amplification, or any sequence library production and/or further reduction method. Relating thereto, in a preferred embodiment the present invention provides a method for analysis of target nucleic acids, the method comprising the following steps:
i. providing a sample wherein target nucleic acids are present in a low amount;
ii. generating a reduced representation library of said target nucleic acids, using the steps of
   - fragmenting said target nucleic acids;
   - ligating adaptors to said fragments;
   - selecting a subset of said adaptor-ligated fragments;
   - simultaneously with or after selecting a subset, amplifying said subset;
iii. massively parallel sequencing said reduced representation library; and
iv. identifying variants in said target nucleic acids by analyzing results obtained by said sequencing.

In a preferred embodiment, no amplification is performed between the provision of said sample and the fragmentation of said target nucleic acids. In another particular embodiment, selection and amplification of said subset is performed simultaneously, e.g. by PCR-amplification. In yet another preferred embodiment, generating a reduced representation library comprises amplifying a subset of fragments which, when combined, comprise only a part of the target nucleic acids.

Any method known to the skilled person can be used for the selection (and optional amplification) of a subset of adaptor-ligated fragments. In a particular embodiment, said selection is performed by PCR amplification using a selective primer as described hereinbefore. In another particular embodiment, said PCR amplification comprising the use of a temperature profile to preferentially amplify fragments of a certain size. E.g. PCR amplification may preferentially amplify small sized fragments.

In other preferred embodiments of the invention any sequence library production and/or further reduction method may be amplicon sequencing libraries produced from DNA following single-cell lysis.

In other preferred embodiments of the invention the method further may comprise the step of deep sequencing of the reduced representation library to assure that each variant position is sampled with high redundancy.

In preferred embodiments of the invention a pipeline for variant calling may be based on the detection of variant allele frequencies in the sequence reads that can be discriminated from sequencing and/or amplification artifacts using for instance a pipeline of sequence alignment, bioinformatics and statistics.

In other preferred embodiments of the invention the variant allele frequencies may be rare variant allele frequencies.

In preferred embodiments of the invention using a pipeline of sequence alignment may be performed using a reference genome.

In other preferred embodiments of the invention the method further may comprise the step of inferring genotype calls from detected variant allele frequencies.

In preferred embodiments of the invention the method further may comprise haplotype assessment or prediction of the at least one, preferably a single, cell's genotype.

In preferred embodiments of the invention amplifying may be performed on any desired part of the genome by rolling circle amplification. Preferably a rolling circle amplication may be performed on the circular mitochondrial DNA.

The methods described in this application can be used/applied to human and animal cells for embryo selection purposes, for genetic studies of heterogeneous tissues consisting of cells with different allelic constitutions (e.g. tumors), or for forensic research. The developed generic methods have immediate applicative value for e.g. preimplantation genetic diagnosis (PGD) of in vitro fertilized human embryos in the clinic, or for animal breeding programs by enabling selection of embryos for multiple (quantitative trait) loci in a single experiment, or for genetic studies of heterogeneous tissues that consist of cells with different allelic constitutions (e.g. tumors), as well as all genetic studies requiring genetic polymorphism typing (such as SNP typing or genetic variant detection by DNA-sequencing) or haplotyping data in general.
In addition, embodiments of the genotyping/haplotyping method of the present invention allow further characterization of drivers of haplotype diversity, primarily meiotic homologous recombination, but also mitotic recombination processes that may occur at elevated frequencies during tumorigenesis. Inter- as well as intra-chromosomal rearrangements in somatic cells alter the sequence of syntenic alleles leading to the potential activation of proto-oncogenes and inactivation of tumor suppressor genes. Hence, such recombinations may initiate tumorigenesis, but may also contribute to tumor progression. Due to this chromosome instability cells within a tumor are heterogeneous and in addition tumor biopsies are contaminated with normal somatic cells. The methods of the present invention advantageously allow to gain more insight in tumor development and recombination processes.

Thus, the methods of the invention are applicable on any cell type. Preferred cells are polar bodies, blastomeres, trophectoderm cells from blastocysts or chorionic villus samples. Preferred genetic material comprises DNA, more particularly cell-free DNA. Preferably the cell-free fetal DNA is from maternal blood, plasma or serum. Both intact fetal cells and fetal cell-free nucleic acids (DNA, RNA) can be identified in maternal blood. The primary source of most fetal cell-free nucleic acids in the maternal circulation is thought to be apoptosis of placental cells. As already mentioned hereinbefore, the methods are applied on a small number of these cell types, i.e. on a few cells, in particular on one or two cells. When applied on trophectoderm said few cells may be selected from 1, 2, 3, 4, 5, 6, 7, 8, 9, 10 or more cells; in particular up to 50 trophectoderm cells.

For the removal of the appropriate at least one cell, the zona pellucida at the cleavage and blastocysts stages can be breeched by mechanical zona drilling, acidified Tyrodes solution or laser. In preferred embodiments of the invention the at least one, preferably a single, cell is a human or animal blastomere.

In particular embodiments, the genetic testing is applied for diagnostic testing, carrier testing, prenatal testing, preimplantation testing, or predictive and presymptomatic testing. In these particular embodiments genetic testing assists to help patients achieve success with assisted reproduction. In another particular embodiment, the methods of the invention are applied for newborn screening. In yet another particular embodiment, the methods of the invention are applied for forensic testing.

In another particular embodiment, the methods of the present invention can be applied for determining the presence of a tumor cell, or for determining minimal residual disease or disease progression. In another particular embodiment, said methods can be applied for determining the risk of developing a tumor or cancer. In a particular embodiment, the methods of the invention are applied on one or more cells suspected of being tumor or cancer cells. In another particular embodiment, the methods of the invention are applied on a fluid sample from a subject suspected of having a tumor or cancer. Preferably, said fluid sample is a blood, plasma or serum sample. In a further embodiment, the methods of the present invention are applied on cell-free tumor DNA. In another preferred embodiment, the methods of the invention are applied on circulating tumor DNA.

In particular embodiments, the methods of the invention apply reduced-representation sequencing and questions about genetic variation are answered by sequencing a small set of genome-wide regions without sequencing the whole genome. Genome library reduction methods applying digestion of the genomic material may use one, two, three, four or more restriction enzymes. The choice of the enzyme may be determined by the marker density required. Most often the genomic DNA is digested with one or more frequently cutting restriction enzymes of choice. The resulting restriction fragments are selected by size and then sequenced producing partial but genome-wide coverage.

Sequencing may apply shotgun sequencing or targeted sequencing. In particular, sequencing refers to massively parallel sequencing, also termed next-generation sequencing. Preferred sequencing methods include pyrosequencing (454), Ion Torrent sequencing, Illumina dye sequencing, etcetera.

Methods according embodiments of the disclosure may be implemented on a computer as a computer-implemented method, or in dedicated hardware, or in a combination thereof. Executable code for a method according to the invention may be stored on a computer program product. Examples of computer program products include memory devices, optical storage devices, integrated circuits, servers, online software, etc. The hardware may comprise a microcontroller or a processor, etc.

In a second aspect, the present disclosure provides a data carrier storing a computer program product according to embodiments of the methods of the present invention. The term "data carrier" is equal to the terms "carrier medium" or "computer readable medium", and refers to any medium that participates in providing instructions to a processor for execution. Such a medium may take many forms, including but not limited to, non-volatile media, volatile media, and transmission media. Non-volatile media include, for example, optical or magnetic disks, such as a storage device which is part of mass storage. Volatile media include dynamic memory such as RAM. Common forms of computer readable media include, for example, a floppy disk, a flexible disk, a hard disk, magnetic tape, or any other magnetic medium, a CD-ROM, any other optical medium, punch cards, paper tapes, any other physical medium with patterns of holes, a RAM, a PROM, an EPROM, a FLASH-EPROM, any other memory chip or cartridge, a carrier wave as described hereafter, or any other medium from which a computer can read. Various forms of computer readable media may be involved in carrying one or more sequences of one or more instructions to a processor for execution. For example, the instructions may initially be carried on a magnetic disk of a remote computer. The remote computer can load the instructions into its dynamic memory and send the instructions over a telephone line using a modem. A modem local to the computer system can receive the data on the telephone line and use an infrared transmitter to convert the data to an infrared signal. An infrared detector coupled to a bus can receive the data carried in the infrared signal and place the data on the bus. The bus carries data to main memory, from which a processor retrieves and executes the instructions. The instructions received by main memory may optionally be stored on a storage device either before or after execution by a processor. The instructions can also be transmitted via a carrier wave in a network, such as a LAN, a WAN or the internet. Transmission media can take the form of acoustic or light waves, such as those generated during radio wave and infrared data communications. Transmission media include coaxial cables, copper wire and fibre optics, including the wires that form a bus within a computer.

In a third aspect, the present disclosure provides in transmission of a computer program product according to the second aspect of the present invention over a network.

In a fourth aspect, the disclosure provides systems for haplotyping at least one cell, whereby the system may comprise, a control unit, said control unit adapted to:
- isolate and lyse the at least one cell,
- amplifying DNA fragments of the least one cell,
- massively parallel (genome-wide) genetic polymorphism typing (genotyping) by deep sequencing a reduced representation library of said amplification product,
- provide a pipeline for variant discovery, genotyping and/or haplotyping.

In an alternative aspect, the disclosure provides systems for haplotyping a single cell, whereby the system may comprise, a control unit, said control unit adapted to:
- isolate and lyse the single cell,
- amplifying DNA fragments of the single cell,
- massively parallel (genome-wide) genetic polymorphism typing (genotyping) by deep sequencing a reduced representation library of said amplification product,
- provide a pipeline for variant discovery, genotyping and/or haplotyping.

In yet another aspect, the disclosure provides systems for haplotyping dual cells, whereby the system may comprise, a control unit, said control unit adapted to:
- isolate and lyse the two cells,
- amplifying DNA fragments of each single cell,
- for each cell, massively parallel (genome-wide) genetic polymorphism typing (genotyping) by deep sequencing a reduced representation library of said amplification product,
- generate a virtual genotype consisting of genetic polymorphism call concordant between the two separately genotyped single cells,
- reconstruct the haplotype of said virtual genotype (or a selection of said virtual genotype)
- provide a pipeline for variant discovery, genotyping and/or haplotyping.

In a particular embodiment, the present disclosure provides a system or a device adapted to perform the embodiments of the invention. Said system or device may comprise one or more control units to control the method steps of the invention. Furthermore, the present invention provides a combination of devices, each device adapted to perform one or more of the method steps of the invention.

In another particular embodiment, the present disclosure provides a system for generating a sequencing library, said system being adapted to receive a sample wherein target nucleic acids are present in a low amount, said system comprising a control unit that controls the generation of a reduced representation sequencing library of said target nucleic acids by controlling the
- fragmenting of said target nucleic acids;
- the ligation of adaptors to said fragments; and
- the selection of a subset of said adaptor-ligated fragments.

In a further embodiment, the present disclosure provides a system for generating a sequencing library, said system comprising one or more control units that control:
- the isolation of a sample comprising low amounts of target nucleic acids; in particular the isolation of a few cells;
- the generation of a reduced representation sequencing library according to the methods of the invention; in particular
   - fragmenting said target nucleic acids,
   - ligating adaptors to said fragments, and
   - selecting a subset of said adaptor-ligated fragments.

In a further embodiment, said one or more control units are further adapted to control sequencing, in particular deep sequencing, of said reduced representation sequencing library.

In another particular embodiment, the present disclosure provides a system for analysis of target nucleic acids, said system being adapted to receive a sample wherein target nucleic acids are present in a low amount, said system comprising one or more control units that control
- the generation of a reduced representation library of said target nucleic acids according to the methods of the invention, and
- sequencing said reduced representation library.

In a particular embodiment, the present disclosure provides a combination of devices comprising:
- a cell isolation device adapted to isolate a few cells; in particular one to twenty cells; and
- a sample processing device adapted to:
   - generate a reduced representation library as described herein, and
   - perform massively parallel sequencing of said reduced representation library.

In addition, the present disclosure provides a combination of devices comprising:
- a cell isolation device adapted to isolate a few cells; in particular one to twenty cells;
- a sample processing device adapted to generate a reduced representation library according to the methods of the invention; and
- a massively parallel sequencing device.

In preferred embodiments the present disclosure advantageously provides methods for high throughput genotyping by sequencing of single cells (Sc GBS).

Embodiments of the present invention provide a generic approach, which can be used to directly identify genetic variations derived from different genomes advantageously unrelated to their size and/or GC content and infers genotypes and/or haplotypes regardless the used high-throughput massive parallel sequence technology. In addition embodiments of the present invention can advantageously have various applicative values, e.g.:
(1) in human or non-human fertility clinics conducting pre-implantation genetic testing on in vitro or in vivo produced pre-implantation embryos,
(2) in animal breeding programs for genomic selection applications,
(3) in genetic test centers analyzing heterogeneous tissues that consist of cells with different allelic constitutions in (e.g. tumors), and
(4) in all genetic studies requiring genome-wide genetic variation detection for genotype and/or haplotype reconstructions.

Embodiments of the present invention provide genome-wide variation discovery and/or typing in at least one, preferably a single cell or a few cells, to infer genotypes and/or haplotypes preferably derived from reduced-representation sequencing data, for instance by using current high-throughput massively parallel sequencing technologies know in the art. Independent of sequencing platform design and chemistry, population variation or genome constitution (e.g. SNP arrays), embodiments of the present invention advantageously provide a cost-efficient, fast and generic strategy. Samples may be pooled before sequencing using different adaptor-linked barcodes making this approach beneficially highly scalable (from low to ultra-deep sequencing) and cost-efficient for applicability in diagnostics.

Ultra-deep sequencing or amplicon sequencing used in embodiments of the invention preferably allows one to detect mutations at extremely low levels, and PCR amplify specific, targeted regions of DNA. This method is preferably used to identify low frequency somatic mutations in cancer samples or discovery of rare variants.

The method, according to preferred embodiments of the invention, can comprise at least one of the following steps with regard to at least one, preferably a single, a few cells or genetic material:
1. In case of cell preparation, said cell preparation preferably comprising;
   a. Isolating at least one cell(s), preferably a single or a few cells, e.g. from in vitro or in vivo generated pre-implantation embryos
   b. Lysing the cells

   In case of genetic material, no cell preparation is required and the genetic material is obtained from an appropriate fluid such as blood, plasma or serum.
2. Amplifying the whole genome preferably using multiple displacement amplification or any (whole-genome) amplification method which e.g. can be based on PCR using for instance (semi-)random primers; or adaptor ligation onto single-cell DNA-fragments and/or universal primers for amplification
   a. In an optional step, whole-genome amplification can be omitted and only desired fractions of the single-cell genome can be amplified, e.g. amplification of mitochondrial sequences specifically using for instance one primer and a rolling circle amplification principle. Rolling circle amplification (RCA) is a molecular amplification method with the unique property of forming concatameric DNA that is composed of thousands of tandemly repeated copies of the initial sequence. Advantageously as few as 150 molecules bound to the surface of microarrays can be detected using RCA. Because of the linear kinetics of RCA, nucleic acid target molecules may be measured with a dynamic range of four orders of magnitude. Such partial genome amplification (PGA) methods advantageously already significantly reduce the complexity of the single-cell genome before massively parallel sequencing of the PGA-products.
   b. In an optional step, whole-genome amplification (WGA) and partial-genome amplification (PGA) can be omitted and preferably a nascent single-cell DNA is preferably immediately processed for GBS.
3. Constructing a reduced representation library (RRL) either for instance by eliminating complex genomic structures (e.g. restriction digestion) or by for instance enrichment of the DNA of interest by partial genome amplification or capture of target sequence including exomes to ensure library size and complexity reduction of the amplified fragments.
   a. In an optional step, preparation of the RRL is preferably omitted and the total single-cell amplification product is can be analyzed via massively parallel sequencing (e.g. amplification of mitochondrial sequences using one specific primer).
   b. In an optional step, ligation of barcoded adaptors sequences can be performed immediately on non-amplified single-cell DNA fragments and preferably subsequently pooled with different samples in an equimolar proportion.
   c. Polymerase Chain Reaction (PCR) amplifying of the library, preferably to size select fragments of e.g. 200-300 bp, preferably avoiding the use of size selection steps (e.g. Caliper Labchip XT, gel-based). Quality control of the library should preferably be done at this step.
4. Massively parallel DNA-sequencing of the library (independent of platform or chemistry).
5. Identification of SNPs and/or variation discovery, preferably done as follows according to embodiments of the invention: reference sequence mapping or de novo local assembly of reads, preferably followed by genotyping of genetic markers using a specific variant calling algorithm/tool, advantageously allowing for amplification bias estimate and likelihood calculation of genotype.
6. Reconstructing the genotypes, preferably with genome location and individual ID.

In an optional step, reconstructing or imputing the haplotype, preferably is based on earlier knowledge or reference data.

In a particular embodiment, the present invention provides methods for analysis of target nucleic acids in two or more samples, the method comprising the following steps:
- providing a first sample wherein target nucleic acids are present in a low amount,
- providing a second sample wherein target nucleic acids are present in a low amount,
- generating a first reduced representation library of the target nucleic acids in said first sample comprising incorporation of a first tag in the fragments in said first reduced representation library,
- generating a second reduced representation library of the target nucleic acids in said second sample comprising incorporation of a second tag in the fragments in said second reduced representation library,
- optionally pooling said first and second reduced representation library,
- massive parallel sequencing said first and second reduced representation library, and
- identifying variants in said target nucleic acids by analyzing results obtained by said sequencing, wherein said variants are identified as being present in said first or second sample using said first or second tag.

Advantageously, incorporation of a first or second tag can easily be performed by using tagged ("barcoded") adaptors.

### Definitions

The term "GBS" as used herein refers to "Genotyping by sequencing a reduced representation library"

The term "Direct GBS" as used herein refers to "Genotyping by sequencing a reduced representation library produced from DNA immediately following cell lysis without an intervening whole- or partial-genome amplification step."

The term "small analyte" as used herein refers to a very small amount of the analyte. Preferred analytes are at least one cell, preferably a few cells, a dual cell, a single cell, or cell-free DNA such as cell-free fetal DNA in maternal fluid.

The term "maternal fluid" as used herein refers to a maternal fluid sample, such as a blood, plasma or serum sample.

The term "genetic testing" as used herein refers to testing to identify variations (disorders, changes) in chromosomes, subchromosomal regions, genes or proteins. Chromosomal variations (e.g. aneuploidy), copy number variations (CNVs), insertions and deletions (INDELs) and single nucleotide polymorphisms (SNPs) are forms of genetic variation. Variant discovery, including aneuploidy or ploidy calling, copy number variation calling, genotyping and/or haplotyping, can help to confirm or rule out a suspected genetic condition or help determine a person's chance of developing or passing on a genetic disorder. Such genetic tests may be useful in for instance newborn screening, diagnostic testing, carrier testing, prenatal testing, preimplantation testing, predictive and presymptomatic testing or forensic testing.

As used herein, variant discovery, variant calling and variant identification are used interchangeably. A "variant" refers to any genetic polymorphism, such as, but not limited to, SNPs, INDELs or CNVs. "Genotyping" as used herein applies to SNP, INDEL or CNV variation typing.

"Genetic material" or "Genetic sample" as used herein refers to chromosomes, DNA, RNA or subunits thereof.

"Aneuploidy" refers to losses and/or gains of individual chromosomes from the normal chromosome set. In the case of a somatic human cell it refers to the case where a cell does not contain 22 pairs of autosomal chromosomes and one pair of sex chromosomes.

The term "Isolating" as used herein refers to obtaining.

"Deep sequencing" as used herein refers to sequencing at a high redundancy. In a preferred embodiment, deep sequencing refers to sequencing with a depth (i.e. average number of reads representing a given nucleotide in the sequencing library) of at least 1x. In a preferred embodiment, deep sequencing refers to a depth of at least 5x, in particular at least 10x, more in particular at least 50x . In another preferred embodiment, fragments in the sequencing library are sequenced with a depth of at least 100x, in particular at least 200x, more in particular at least 300x. In a further embodiment, so-called ultra-deep sequencing is performed, indicating sequencing depths of at least 500x, in particular at least 750x, more in particular at least 100x.

As is evident from the description of the invention herein, the methods of the present invention are preferably applied to samples containing low amounts of target nucleic acids, also referred to as genetic material. In particular, said genetic material of interest is either present within one or a few target cells, or as free circulating material in the sample. Thus in a particular embodiment, said sample contains one or a few target cells. In a further embodiment, said sample contains one target cell. In another embodiment, said sample contains a few target cells, in particular 1 to 30, more in particular 1 to 20, target cells. For example, 1-15, 1-10, 1-8, 1-7, 1-6, 1-5, 1-4, 1-3, one or two target cells. In another particular embodiment, target nucleic acids are present in an amount of 2 ng or less in said sample, in particular 1 ng or less, more in particular 0.5 ng or less. In another particular embodiment, target nucleic acids are present in an amount of 250 pg or less in said sample; in particular 200 pg or less; more in particular 150 pg or less. In another particular embodiment, said target nucleic acids are present in an amount of 100 pg or less; in particular in an amount of 50 pg or less; more in particular in an amount of 30 pg or less. In another particular embodiment, said target nucleic acids are cell-free, circulating nucleic acids. For example, circulating cell-free fetal DNA from a maternal sample, or circulating tumor DNA from a patient sample. While genetic material (e.g. maternal DNA) may be abundant in such samples, target DNA (e.g. fetal DNA) is present in only very limited amounts. In a particular embodiment, target nucleic acids are present as cell-free nucleic acids in a fluid sample. In particular, said cell-free nucleic acids are present in a fluid sample comprising additional (non-target) nucleic acids. In a particular embodiment, said sample comprises a mixture of target and non-target nucleic acids. Preferably, said target nucleic acids are present in an amount between 0.1 and 20% of said non-target nucleic acids. In another particular embodiment, said sample comprises a mixture of target and non-target nucleic acids, wherein said target nucleic acids are present in an amount of 700 ng or less, in particular 500ng or less, more in particular 300 ng or less. In a further embodiment, 200 ng or less, in particular 100 ng or less, more in particular 50 ng or less. In yet another embodiment, said sample comprises cell-free nucleic acids, wherein said cell-free nucleic acids are present in an amount as defined hereinabove.

In a particular embodiment, providing a sample comprising low amounts of target nucleic acids comprises isolating one or a few target cells. The methods of the invention may further comprise lysing one or a few target cells.

The sample is preferably obtained from a eukaryotic organism, more in particular of a mammal. In a further preferred embodiment, said sample is from non-human animal (hereinafter also referred to as animal) origin or human origin. In a particular embodiment, said animal is a domesticated animal or an animal used in agriculture, such as a horse or a cow. In a further particular embodiment, said animal is a horse. In another particular embodiment, said sample is of human origin. In yet another particular embodiment, said sample is obtained from a pregnant woman. In another embodiment, said sample is obtained from a patient suspected from having a tumor or cancer. In another particular embodiment, said cell is a eukaryotic cell, in particular a mammalian cell. In a more particular embodiment, the origin of said cell is as described according to preferred embodiments regarding the sample origin as described above. In another particular said target nucleic acids are of eukaryotic origin, in particular of mammalian origin. In a more particular embodiment, said target nucleic acids are as described according to the preferred embodiment regarding the sample origin. Relating thereto, in a preferred embodiment, said target nucleic acids originate from an embryo or a fetus. In another preferred embodiment, said target nucleic acids originate from a (suspected) cancer or tumor cell.

"Genome-wide" as used herein means that the methods are applied to and provide information on sequences throughout the genome. In particular, the methods of the present invention provide information regarding all chromosomes for which at least fragments are present in the sample. In a particular embodiment, "genome-wide" refers to information regarding at least one variant per 100 Mb, in particular at least one variant per 10 Mb, in particular at least one variant per 1Mb throughout the genome. In a further embodiment, it is meant at least one variant per window of 100 Mb, in particular at least 1 variant per window of 50 Mb, more in particular at least one variant per window of 10 Mb throughout the genome. In another particular embodiment, genome-wide refers to information regarding at least one variant per window of 1 Mb.

### Brief description of the drawings

Further features of the present invention will become apparent from the examples and figures, wherein:
Fig. 1 illustrates accuracy of WGA nucleotide-copying method used in embodiments of the present invention.
Figure 2: Size distribution of the genomic library of 1 horse after restriction digestion with *Apekl.* X-axis shows the fragment length in basepairs and the Y-axis shows the fluorescence units. Two peaks at 35bp and 10380bp refer to lower and upper marker, respectively.
Figure 3: Size distribution of the genomic library of 1 horse after sequencing with a peak around 110 bp. X-axis shows the fragment length in basepairs and the Y-axis shows the number of fragments called at that particular length.
Figure 4: This figure shows an improvement of the complexity reduction of the horse genome when using the standard versus the selective method. The black boxes indicate the average sample (meaning the average of 56 samples) sequenced with the standard method. The transparent boxes indicate the average sample sequenced with the selective method. The Y-axis shows the number of reads.

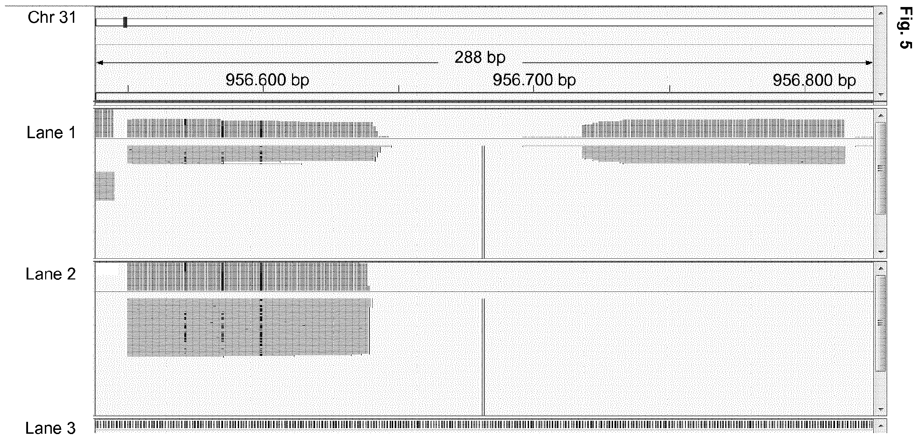

Figure 5: This snapshot of the IGV browser zooms into of a particular region of 288 bp on chromosome 31. The upper box indicates the chromosome location and the genomic size of the window. Lane 1 visualizes the pooled data of the 56 samples sequenced via the standard method whereas lane 2 visualizes the pooled data of the 56 samples sequenced via the selective method. Lane 3 shows the locations of the recognition sites of the *Apekl* enzyme. The black bars in lane 1 and 2 indicate the presence of a nucleotide difference with the reference sequence (EquCab2). Each horizontal bar/dot in lanes 1 and 2 refer to a sequence difference in one individual sample.

### Detailed description of preferred embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

The term "comprising", used in the claims, should not be interpreted as being restricted to the means listed thereafter; it does not exclude other elements or steps. Thus, the scope of the expression "a system comprising means A and B" should not be limited to systems consisting only of components A and B. It means that with respect to the present invention, the relevant components of the system are A and B.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In the drawings, like reference numerals indicate like features; and, a reference numeral appearing in more than one figure refers to the same element. The drawings and the following detailed descriptions show specific embodiments of the system and method for high-throughput genotyping by sequencing of single cells.

Embodiments of the invention advantageously provide a method whereby at least a single cell DNA-isolation, with or without (n/mtDNA) amplification, can be combined with a complexity reduction of the target, e.g. single cell, DNA product, PCR-based amplification and next generation sequencing to produce a set of markers for genotyping and haplotyping complete genomes, or parts of it, of one to multiple cells. In addition to the novel combination of those steps, other embodiments of the present invention advantageously provide a novel method to filter by for instance bioinformatics/statistical means the artifacts generated by any whole- or partial-genome amplification (WGA or PGA respectively) or PCR of (reduced representation) sequencing library as well as sequencing method.

The advent of next generation sequencing (NGS) technologies have revolutionized the way biologists produce, analyze and interpret data. Although NGS platforms provide a cost-effective way to discover genome-wide variants from a single experiment, variants discovered by NGS need follow up validation due to the high error rates associated with various sequencing chemistries, in addition molecular analysis of single cells is challenging due to the low amounts of DNA available. Advantageously whole exome sequencing has been proposed as an affordable option compared to whole genome runs but it still requires follow up validation of all the novel exomic variants. Customarily, a consensus approach is used to overcome the systematic errors inherent to the sequencing technology, alignment and post alignment variant detection algorithms. However, the aforementioned approach warrants the use of multiple sequencing chemistry, multiple alignment tools, multiple variant callers which may not be viable in terms of time and money for individual investigators with limited informatics know-how. Biologists often lack the requisite training to deal with the huge amount of data produced by NGS runs and face difficulty in choosing from the list of freely available analytical tools for NGS data analysis. Hence, there is a need to customize the NGS data analysis pipeline to preferentially retain true variants by minimizing the incidence of false positives and make the choice of right analytical tools easier. To this end, embodiments of the present invention advantageously provide methods which can overcome these drawbacks, by providing advanced data correction methods, resulting in efficient and robust results.

In addition, current single-cell genotyping problems, mainly due to allele drop out and drop in and/or preferential allele amplification bias following single-cell DNA-amplification methods can be largely overcome by deep sequencing according to preferred embodiments of the present invention to assure that each base pair is sampled with high redundancy. Embodiments of the method and related bioinformatic means advantageously enable one to identify those (rare) variants.

A method according to embodiments of the invention can comprise at least one of the following steps:

### (i) Isolate single cells, DNA extraction and whole genome amplification (WGA).

Briefly, when single or more cells get isolated by either picking of facsing cells, their nuclei containing the DNA and the mitochondrial DNA may then be amplified after cell lysis via genome wide amplification methods based on Multiple Displacement Amplification (MDA) or PCR-based genome-wide amplification. The result is a collection of fragments (large or small depending on the WGA-method used). This collection will then be processed for genotyping by sequencing (GBS) using restriction enzymes to construct a representation library (RRL) for high-throughput massive parallel sequencing. In an optional step, WGA of the single-cell DNA is omitted and only particular or desired fractions of the single-cell genome are amplified. These partial genome amplification (PGA) methods already significantly reduce the complexity of the single-cell genome before massively parallel sequencing / GBS. In another optional step, WGA and PGA of the single-cell DNA are omitted, and the single-cell DNA following cell-lysis is immediately processed for GBS (i.e. direct GBS).

### (ii) In silico digestion and enzyme selection.

Restriction Enzymes can be selected preferably based upon following criteria:
(1) predicted fragments length/nr of restriction sites,
(2) the proportion of overlap with repetitive elements/methylation sites,
(3) the putative SNP content,
(4) the frequency of enzyme cutting,
(5) predicted coverages of single-cell whole-genome amplification methods. Embodiments of the present invention advantageously provide means to construct and integrate 'zero-coverage' maps of a genome, i.e. maps highlighting those bases that are recurrently missed by sequences of single-cell amplification products.

Each single-cell WGA-library sequenced for a particular amount of bases preferably produces a WGA-characteristic pattern of sequence coverage breadth and depth across the reference genome. E.g. single-cell PCR-based sequences recurrently miss more parts of the genome than sequences of multiple displacement amplified (MDAed) cells, but loci covered by single-cell PCR-based sequences are often covered deeper when compared to sequences of MDAed cells although both have been sequenced for the same amount of bases.

Preferred embodiments of the invention provide a combination of Restriction Enzymes which preferably can be chosen to perform double or more digests to increase SNP discovery rates and thus increase the overall sensitivity of genotyping assays. When the enzymes are chosen, a digest is preferably prepared on the WGA samples followed by a fragment selection based upon size.

### (iii) Library construction and DNA sequencing

Next a purification of the chosen fragments is preferably performed followed by the addition of adaptors with (preferably) a single nucleotide overhang.

### (iv) SNP calling (e.g. identification and/or typing) and data handling

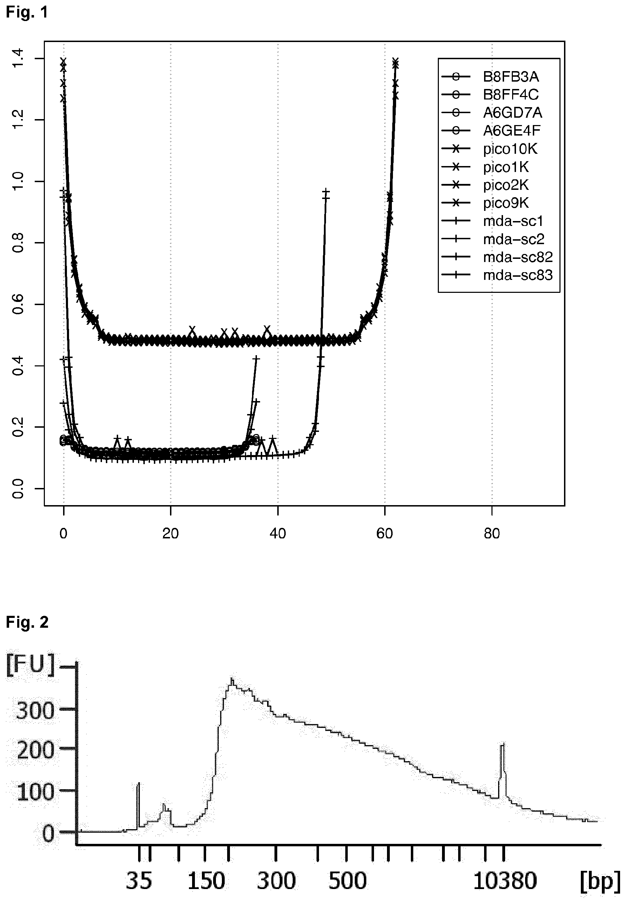

Results of using a method according to embodiments of the invention advantageously demonstrate that sequencing of single-cell WGA-products enables to determine digital frequencies of both alleles of a genetic marker (SNP, Indel...) in the WGA-DNA. This has the advantage that e.g. SNPs in single cells may be typed more accurately when compared to conventional methods that use e.g. SNP-arrays. Indeed, preferential amplification of one allele of a heterozygous SNP will for instance result in a homozygous SNP-call when analyzed on a SNP-array because of the overwhelming signal of this preferentially amplified allele on the SNP-probes of the array. In contrast, in the sequencing approach the heterozygous SNP can be called with much more accuracy and confidence because e.g. hundreds to thousands of sequence reads report the preferentially amplified allele, but also a minority of reads will report the other allele of the SNP. Hence, this insight will allow a genotyping algorithm according to embodiments of the invention (see below) to tilt with statistical confidence the single-cell SNP-call towards a correct heterozygous instead of a false homozygous call. Similar rules apply when single-cell DNA is processed via PGA or direct GBS without intervening WGA/PGA. Although nucleotide substitutions can be identified in single-cell WGA-sequences, WGA-polymerases do not copy every base correctly during the amplification. Those errors may be mistaken for genuine nucleotide substitutions in the cell's genome. To investigate the base-fidelity of WGA-polymerases, the mismatch frequency of bases (having a base-call quality of ≥30) has been charted to the reference genome across the entire length of reads (having a mapping quality of ≥30). Strikingly, the mismatch frequency was significantly higher following single-cell PCR-based WGA-sequencing than following single-cell MDA-based or non-WGA DNA-sequencing (as illustrated in Fig. 2 which shows a two-tailed Kolmogorov-Smirnov test, with p-values < 2.2e-16), suggesting that certain PCR-based polymerase(s) make significantly more nucleotide copy-errors. The MDA's phi29 polymerase applies 3'->5' proofreading exonuclease activity and preliminary results indicate that the MDA-sequence error-rate is very low and almost comparable to conventional non-WGA DNA-sequencing when applying base-call and mapping qualities of 30 or more as shown in Fig. 2.

Figure 1 moreover illustrates nucleotide mismatch frequency with the hg19-reference genome at each base of the read. Only bases with a base-call quality of 30 or more in reads having a minimum mapping quality of 30 were considered. It is clear that the single-cell PCR-based WGA-method introduces significantly more WGA-nucleotide errors than single-cell MDA-WGA and non-WGA DNA sequencing.

Besides the fidelity of single-cell WGA-polymerases, also the precision of GBS-PCR polymerases and sequence chemistry reactions (e.g. bridge-PCR polymerases) have to be taken into account in the methods for genotyping following single-cell (WGA/PGA-)GBS.

There are two main approaches for interpreting the sequence reads resulting from a single-cell (WGA/PGA-)GBS method according to preferred embodiments of the invention:
(1) Genotyping of the cells for a known set of polymorphic markers (SNPs, Indels,...) or DNA-mutations covered by the single-cell (WGA/PGA-)GBS reads. Although the workflow can be applied for any nucleotide genetic variant that one wishes to genotype in the resulting single-cell sequences, current known SNP positions in the human genome hg19 can for instance be retrieved from databases as dbSNP or from the 1000 Genomes project. Similar databases exist for other species. The physical positions of the nucleotide genetic variants are preferably applied to generate pileups of the bases covering a particular position. Although there may be various algorithmic methods to achieve this, moreover preferred embodiments of the invention provides a pipeline based on e.g. Burrows Wheeler Alignment (BWA), SAMtools, Perl and R-scripts. In brief, for each position that is interrogated by the algorithm according to embodiments of the invention, a list of the amounts of A-, C-, G- and T-bases covering that position is preferably generated, the reference allele is preferably identified as well as all putative alternative (variant) alleles for that position. Thresholds on read mapping quality, base call quality, start and end of reads (e.g. Fig. 2 indicates that the first and last bases of sequence reads should be omitted from the analysis as they contain more mismatch errors with the reference genome) can be applied to increase accuracy at a cost of coverage. If the reference and alternative allele of the SNP are known (e.g. cytosine and thymidine bases for the major and minor allele of the SNP in the general population respectively), the algorithm according to preferred embodiments of the invention advantageously will return the amount of sequence reads carrying the reference allele (e.g. 20 reads reporting a C-base at that position in the WGA-sequence) and similarly for the alternative allele (e.g. 980 reads reporting a T-base at that position in the WGA-sequence). Subsequently, for instance by using statistical testing these digital allelic counts can be evaluated to be significantly different from a situation where sequence error and/or putative WGA nucleotide-copy error would lead to a similar observation if the underlying SNP is homozygous. Based on subsequent P-value thresholds, heterozygous, homozygous and SNP-No calls may be established. Considering that WGA allele drop-out and preferential amplification artifacts often encompass multiple kilobases, SNPs or nucleotide genetic variants in the haplotype of a near variant are expected to have similar allelic variant frequencies in the single-cell WGA-GBS product. By applying this principle, according to preferred embodiments of the invention, advantageously the accuracy in the final genotype calls are further increased. Similar rules apply when single-cell DNA would undergo PGA-GBS or direct GBS without intervening WGA. For direct GBS, single-cell DNA was immediately digested following lysis, adaptors were ligated, DNA-fragments amplified by PCR, size-selected and the amplicons would be massively parallel sequenced. In this process, also allele amplification bias as well as nucleotide copy errors will be introduced when started from a single cell. Hence, the same algorithmic pipelines, according to embodiments of the invention, can be applied. As the algorithms, according to embodiments of the invention, enable detecting variant alleles with (ultra) low frequencies in the sequences, this pipeline has tremendous value for the detection of (ultra) low-grade genetic mosaicism in deep-sequenced samples as well.
(2) De novo discovery of genetic variants in the cell.

The principles presented above may be applied, according to embodiments of the invention, to all bases covered by the single-cell (WGA/PGA-)GBS for de novo discovery of SNPs in single-cell (WGA/PGA-)GBS products. In addition, these pipelines, according to preferred embodiments of the invention, may be supplemented with standard genetic variant callers (e.g. SAMtools with BCFtools, SOAPsnp, GATK, ...), but because of discrepancies in the frequencies of both alleles of a SNP in the single-cell amplification sequences, as well as WGA/PGA-GBS sequence errors, off-the-shelf available variant callers may produce less accurate single-cell genotypes.

### Examples

### Example 1: SNP identification via Genotyping-By-Sequencing (GBS) in Arabian horse

The aim is to determine the genetic diversity within the Arabian purebred horses based on large scale SNP identification using GBS. Hereto, we collected 56 blood samples. DNA extractions were done with puregene kit (Qiagen). Sample concentrations were checked with the nanodrop and fragmentation was checked on agarose gel.

*In silico* digestion based on the EquCab2 reference sequence using *Apekl* was performed using custom Perl/BioPerl scripts and predicted 2,937,656 fragments <=500bp or 3,766,233 fragments <=1000bp. This number reflects the efficiency of the genome complexity reduction. However this does not takes methylation patterns into consideration.

DNA Libraries were prepared as described (Elshire et al. PLoS One. 2011 6(5):e19379. doi: 10.1371/journal.pone.0019379) with minor modifications. Restriction enzyme *Apekl* was used to reduce the genome complexity per sample. *Apekl* is a type II restriction endonuclease that recognizes the DNA target sequence 5'-G^CWGC-3' (where W=A or T) and cleaves after the first G to produce fragments with three-base 5'-overhangs. The adapters comprised a set of 56 different barcode-containing adapters and a common adapter and had a concentration of 0.3 ng/µl instead of 0.6 ng/µl. quality control was done for 4 samples, horse 1,2,9 and 10. Fragment size and the presence of adaptor dimmers were determined via the Agilent bioanalyzer 2100 (figure 2). After determining the concentration of the samples via a picogreen test, the library was pair-end sequenced on one lane on the Illumina HiSeq2000.

The FASTQ Illumina DNA sequences were processed via our data-analysis pipeline. With custom scripts data were sorted by sample based on the inline barcode (first 6-8bp of read1). After trimming the reads were aligned with BWA v0.6.2 to EquCab2 and regions with a peak coverage >5 X identified with SNIFER and custom scripts.

Sequence results showed on average 1,8 million reads per sample and on average 1X coverage per sample. Table 1 provides an overview of the data generated after sequencing the standard library of 56 Arabian horses. The sample number is shown in column 1. Column 2 shows the number of raw reads per sample, column 3 shows the processed reads per sample counting all region per sample larger than 80bp.

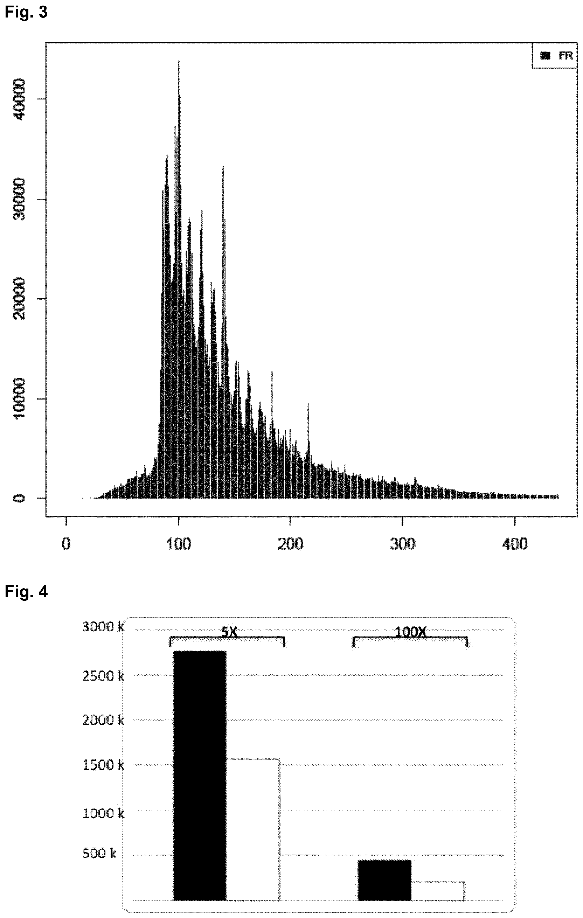

Fragments size distributions of those samples with *Apekl* showed a similar pattern amongst all samples (figure 3). The bam files of all 56 samples were combined and uploaded in the Integrative genomic viewer (IGV). SNPs were analysed by visual inspection (figure 5).

**Table 1:**

| | Raw reads | Processed reads |
|---|---|---|
| | total count | count>80bp |
| 1 | 2505434 | 1582990 |
| 2 | 2844952 | 1809662 |
| 3 | 1790474 | 1132522 |
| 4 | 735215 | 458867 |
| 5 | 3276748 | 2101719 |
| 6 | 2558348 | 1625285 |
| 7 | 2858394 | 1799838 |
| 8 | 2610522 | 1651114 |
| 9 | 2658906 | 1661994 |
| 10 | 2321770 | 1496646 |
| 11 | 3229270 | 2047758 |
| 12 | 1760285 | 1109438 |
| 13 | 1392134 | 878969 |
| 14 | 3270777 | 2154840 |
| 15 | 3354984 | 2199428 |
| 16 | 2742378 | 1759003 |
| 17 | 1167670 | 729718 |
| 18 | 1507787 | 910192 |
| 19 | 799647 | 533114 |
| 20 | 1373434 | 884782 |
| 21 | 1113017 | 708423 |
| 22 | 765382 | 470352 |
| 23 | 154144 | 96367 |
| 24 | 334883 | 200191 |
| 25 | 2831872 | 1780018 |
| 26 | 2856180 | 1813744 |
| 27 | 1889402 | 1141160 |
| 28 | 487088 | 294142 |
| 29 | 1381170 | 909013 |
| 30 | 3267380 | 2118613 |
| 31 | 897341 | 585076 |
| 32 | 611723 | 389776 |
| 33 | 2758005 | 1806251 |
| 34 | 3654815 | 2487642 |
| 35 | 2299255 | 1565585 |
| 36 | 2640480 | 1765888 |
| 37 | 531810 | 349391 |
| 38 | 1740781 | 1165509 |
| 39 | 1172703 | 778117 |
| 40 | 153333 | 100180 |
| 41 | 2368131 | 1580705 |
| 42 | 1582386 | 1048634 |
| 43 | 3178144 | 2162268 |
| 44 | 1911276 | 1253344 |
| 45 | 895756 | 595325 |
| 46 | 1170332 | 778099 |
| 47 | 1324443 | 885272 |
| 48 | 134803 | 89902 |
| 49 | 2299009 | 1531017 |
| 50 | 3403674 | 2320288 |
| 51 | 1421098 | 953557 |
| 52 | 1436544 | 975807 |
| 53 | 1673991 | 1134550 |
| 54 | 848254 | 556281 |
| 55 | 413481 | 278444 |
| 56 | 274165 | 178610 |
| total | 100635380 | 65375420 |
| average | **1797060** | **1167418** |

### Example 2: Further reduction improvement of genome complexity using a selective primer

In addition to the above reduced representation library (further referred to as "standard" library) generation using the *Apekl* restriction enzyme and the sample set of the same 56 Arabian horses, we've reduced genome complexity further by using a selective primer. This selective primer covers the entire common adapter, the 3' restriction site and extends 2 bases into the insert region. Due to the 2 selective bases at the 3' end of the primer, only a subset of adaptor-ligated fragments is amplified.
selective reverse primer (5'-3'): 
CAAGCAGAAGACGGCATACGAGATCGGTCTCGGCATTCCTGCTGAACCGCTCT
TCCGATCTCAGCAC
standard reverse primer (5'-3'): 
CAAGCAGAAGACGGCATACGAGATCGGTCTCGGCATTCCTGCTGAACCGCTCT
TCCGATCT
common forward primer (5'-3'):

AATGATACGGCGACCACCGAGATCTACACTCTTTCCCTACACGACGCTCTTCCG
ATCT

Furthermore, the library preparation was single-end sequenced on a single lane of an Illumina HiSeq2500. Raw sequence reads were processed similar to the above pipeline. Proper quality control was performed to check the correct organisation of the barcode and the restriction site. Poor quality reads, not confirming to our standards, were discarded. Overall, the results show a reduction by half of the genomic complexity in the selective library compared to this of the standard library (figure 4) and an improvement of the average coverage up to 7X sequencing depth.

SNP identification was done similar to the above example and subsequently visualised in the integrative genomic viewer (IGV) (figure 5). The efficiency of the primer is shown as there are fewer regions called in the selective than in the standard library.

### Example 3: Multi cell and Single cell Genotyping-By-Sequencing

A skin biopt of a male horse was taken and cultured in a standard incubator at 37°C and 5% CO2. Fibroblasts of large T175 falcon flask were cultivated, washed and DNA extracted using the blood and tissue kit (Qiagen). The concentration was checked via the nanodrop and DNA fragmentation was checked on agarose gel.

From the same cell line, a single fibroblast was used for further downstream processing. The cell was lysed and DNA amplified according to WO2011/157846.

Library preparations were done using *Pstl* restriction enzyme and further processed similar as the procedure in example 1. *Pst1* was predicted to generate 968,569 fragments in the horse genome (The EquCab2 reference sequence) whereas *ApeKI* 4461178 fragments in total. Since we wanted to maximise the sequencing power, we decided to test the *PstI* digestion on the horse genome. The *PstI* enzyme recognises following sequence CTGCA^G and is methylation sensitive. Further *in silico* predictions estimated 238405 fragments and 388822 fragments smaller than 500bp and 1000bp, respectively.

Sequencing was done of both multicell and single cell on an Illumina HiSeq2000. This resulted in 52K paired-end 100bp reads for the multicell sample and 144K for the single cell sample. Sequence data were processed as described in Example 1. The coverage analyses revealed 15K and 19K regions with a depth of at least 5x, in respectively the multicell and single cell sample, of which 2585 regions were overlapping between both samples. The later is within the expectations given that the total number of predicted regions will be in the range of 250K of which we only observed less than 10% because of the low amount of bases sequenced per sample. Despite a low amount of bases is sequenced per sample, it can lead to local deep-sequencing coverage (e.g. > 5x in this example) by applying the RRL. Samtools v 0.1.17 was used for snp calling in both samples. The positions for which a snp call was observed in both samples were 99% concordant.

## Claims

1. A method for analysis of target nucleic acid, the method comprising the following steps:
i. in a sample that has been provided and wherein target nucleic acids are present in an amount of 100 pg or less, wherein said target nucleic acids originate from an embryo or fetus or from a cancer or tumor cell,
ii. generating a reduced representation library of said target nucleic acids by a method comprising
• fragmenting said target nucleic acids using one or more restriction enzymes;
• ligating adaptors to said fragments; and
• selecting a subset of said adaptor-ligated fragments based on the size of said fragments,
iii. massively parallel sequencing said reduced representation library, and
iv. identifying variants in said target nucleic acids by analyzing results obtained by said sequencing.

2. The method of any one of the previous claims, wherein said selecting a subset is performed using PCR-amplification.

3. The method of any one of the previous claims, wherein said selecting a subset includes PCR amplification using a selective primer.

4. The method of claim 1, wherein generating a reduced representation library comprises amplifying a subset of fragments which, when combined, comprise only a part of the target nucleic acids.

5. The method of claim 1, further comprising
v. constructing a genotype and/or haplotype based on identified variants in said target nucleic acid.

6. The method of claim 1, further comprising
v. identifying a genetic aberration in said sample based on identified variants in said target nucleic acid.

7. The method of claim 1, wherein providing a sample comprises isolating one or a few target cells.

8. The method of claim 7, wherein providing a sample further comprises lysing said one or few target cells.

9. The method of claim 1, further comprising whole genome amplification (WGA) of said target nucleic acids.

10. The method of claim 1, wherein sequencing said reduced representation library assures that each variant position in said library is sampled with high redundancy.

11. The method of any one of the previous claims, wherein said generating a reduced representation library reduces the complexity at least 5 times.

12. The method of any one of the previous claims, wherein said sequencing is performed with a depth of at least 5x.

## Patentansprüche

1. Ein Verfahren zur Analyse einer Ziel-Nukleinsäure, wobei das Verfahren die folgenden Schritte umfasst:
i. in einer Probe, die bereitgestellt wurde und in der Ziel-Nukleinsäuren in einer Menge von 100 pg oder weniger anwesend sind, wobei die Ziel-Nukleinsäuren von einem Embryo oder Fötus oder von einer Krebs- oder Tumorzelle stammen,
ii. Erstellen einer Reduced-Representation-Library der Ziel-Nukleinsäuren durch ein Verfahren, umfassend:
• Fragmentieren der Ziel-Nukleinsäuren unter Einsatz von einem oder mehreren Restriktionsenzymen;
• Binden von Adaptern an die Fragmente; und
• Auswählen eines Teilsatzes der an den Adapter gebundenen Fragmente auf Grundlage der Größe der Fragmente,
iii. massives Parallelsequenzieren der Reduced-Representation-Library, und
iv. Identifizieren von Varianten in den Ziel-Nukleinsäuren durch Analysieren der durch das Sequenzieren erhaltenen Ergebnisse.

2. Das Verfahren nach irgendeinem der vorigen Ansprüche, wobei das Auswählen eines Teilsatzes unter Einsatz von PCR-Amplifikation durchgeführt wird.

3. Das Verfahren nach irgendeinem der vorigen Ansprüche, wobei das Auswählen eines Teilsatzes PCR-Amplifikation unter Einsatz eines selektiven Primers einschließt.

4. Das Verfahren nach Anspruch 1, wobei das Erstellen einer Reduced-Representation-Library das Amplifizieren eines Teilsatzes von Fragmenten umfasst, die, wenn kombiniert, nur einen Teil der Ziel-Nukleinsäuren umfassen.

5. Das Verfahren nach Anspruch 1, ferner umfassend:
v. Aufbauen eines Genotyps und/oder Haplotyps basierend auf identifizierten Varianten in der Ziel-Nukleinsäure.

6. Das Verfahren nach Anspruch 1, ferner umfassend:
v. Identifizieren einer genetischen Abweichung in der Probe basierend auf identifizierten Varianten in der Ziel-Nukleinsäure.

7. Das Verfahren nach Anspruch 1, wobei das Bereitstellen einer Probe das Isolieren von einer oder einigen Zielzellen umfasst.

8. Das Verfahren nach Anspruch 7, wobei das Bereitstellen einer Probe ferner das Lysieren der einen oder einigen Zielzellen umfasst.

9. Das Verfahren nach Anspruch 1, welches ferner Genomamplifikation (whole genome amplification, WGA) der Ziel-Nukleinsäuren umfasst.

10. Das Verfahren nach Anspruch 1, wobei das Sequenzieren der Reduced-Representation-Library sicherstellt, dass jede Variantenposition in der Reduced-Representation-Library mit hoher Redundanz eprobt wird.

11. Das Verfahren nach irgendeinem der vorigen Ansprüche, wobei das Erstellen einer Reduced-Representation-Library die Komplexität mindestens 5 Mal reduziert.

12. Das Verfahren nach irgendeinem der vorigen Ansprüche, wobei das Sequenzieren mit einer Tiefe von mindestens 5x durchgeführt wird.

## Revendications

1. Procédé d'analyse d'un acide nucléique cible, le procédé comprenant les étapes suivantes :
i. dans un échantillon qui a été fourni et
dans lequel des acides nucléiques cibles sont présents en une quantité de 100 pg ou moins, dans lequel lesdits acides nucléiques cibles proviennent d'un embryon ou d'un fœtus ou d'une cellule cancéreuse ou tumorale,
ii. la génération d'une banque de représentation réduite desdits acides nucléiques cibles par un procédé comprenant
• la fragmentation desdits acides nucléiques cibles en utilisant une ou plusieurs enzymes de restriction ;
• la ligature d'adaptateurs auxdits fragments ; et
• la sélection d'un sous-ensemble desdits fragments ligaturés à des adaptateurs sur la base de la taille desdits fragments,
iii. le séquençage massivement parallèle de ladite banque de représentation réduite, et
iv. l'identification de variants dans lesdits acides nucléiques cibles en analysant les résultats obtenus par ledit séquençage.

2. Procédé selon l'une quelconque des revendications précédentes, dans lequel ladite sélection d'un sous-ensemble est effectuée en utilisant une amplification par PCR.

3. Procédé selon l'une quelconque des revendications précédentes, dans lequel ladite sélection d'un sous-ensemble inclut une amplification par PCR en utilisant une amorce sélective.

4. Procédé selon la revendication 1, dans lequel la génération d'une banque de représentation réduite comprend l'amplification d'un sous-ensemble de fragments qui, quand ils sont combinés, comprennent uniquement une partie des acides nucléiques cibles.

5. Procédé selon la revendication 1, comprenant en outre
v. la construction d'un génotype et/ou d'un haplotype sur la base de variants identifiés dans ledit acide nucléique cible.

6. Procédé selon la revendication 1, comprenant en outre
v. l'identification d'une aberration génétique dans ledit échantillon sur la base de variants identifiés dans ledit acide nucléique cible.

7. Procédé selon la revendication 1, dans lequel la fourniture d'un échantillon comprend l'isolement d'une ou de quelques cellules cibles.

8. Procédé selon la revendication 7, dans lequel la fourniture d'un échantillon comprend en outre la lyse desdites une ou quelques cellules cibles.

9. Procédé selon la revendication 1, comprenant en outre une amplification de génome entier (WGA) desdits acides nucléiques cibles.

10. Procédé selon la revendication 1, dans lequel le séquençage de ladite banque de représentation réduite assure que chaque position de variant dans ladite banque est échantillonnée avec une redondance élevée.

11. Procédé selon l'une quelconque des revendications précédentes, dans lequel ladite génération d'une banque de représentation réduite réduit la complexité au moins par 5.

12. Procédé selon l'une quelconque des revendications précédentes, dans lequel ledit séquençage est effectué avec une profondeur d'au moins 5x.
